# EUROPEAN PATENT SPECIFICATION

(11) **EP 3 495 385 B1**
(45) Date of publication and mention of the grant of the patent: **06.08.2025**
(21) Application number: 18208034.1
(22) Date of filing: 14.12.2011
(51) Int. Cl.: C07K 14/755, C12N 7/06, A61K 35/37, A61L 2/00

(54) **VIRAL INACTIVATION USING IMPROVED SOLVENT-DETERGENT METHOD**
VIRALE INAKTIVIERUNG MIT VERBESSERTEM LÖSUNGSMITTEL-DETERGENS-VERFAHREN
INACTIVATION VIRALE AU MOYEN D'UN PROCÉDÉ SOLVENT-DÉTERGENT AMÉLIORÉ

(30) Priority: 15.12.2010 US 42351210 P
(43) Date of publication of application: 12.06.2019
(62) Divisional of application: 11808044.9
(73) Proprietor: Takeda Pharmaceutical Company Limited, Osaka-shi, Osaka (JP)
(72) Inventor: Felgenhauer, Martin, 1180 Vienna (AT); Mison, Dominique, 17340 Chatelaillon-Plage (FR); Montandon, Frederic, 25130 Villers-le-Lac (FR); Farcet, Maria, 1160 Vienna (AT)
(74) Representative: Hoffmann Eitle

(56) References cited:
- HERBERT O. DICHTELMÜLLER ET AL: "Robustness of solvent/detergent treatment of plasma derivatives: a data collection from Plasma Protein Therapeutics Association member companies", TRANSFUSION, vol. 49, no. 9, 1 September 2009 (2009-09-01), pages 1931 - 1943, XP055020543, ISSN: 0041-1132, DOI: 10.1111/j.1537-2995.2009.02222.x
- ROBERTS P L ET AL: "Virus inactivation in a factor VIII/VWF concentrate treated using a solvent/detergent procedure based on polysorbate 20", BIOLOGICALS, ACADEMIC PRESS LTD., LONDON, GB, vol. 37, no. 1, 1 January 2009 (2009-01-01), pages 26 - 31, XP025771406, ISSN: 1045-1056, [retrieved on 20081010], DOI: 10.1016/J.BIOLOGICALS.2008.08.003
- ROBERTS ET AL: "Virus inactivation by solvent/detergent treatment using Triton X-100 in a high purity factor VIII", BIOLOGICALS, ACADEMIC PRESS LTD., LONDON, GB, vol. 36, no. 5, 1 September 2008 (2008-09-01), pages 330 - 335, XP024527519, ISSN: 1045-1056, [retrieved on 20080902], DOI: 10.1016/J.BIOLOGICALS.2008.06.002
- HOLGER SEITZ ET AL: "Comparable Virus Inactivation by Bovine or Vegetable Derived Tween 80 During Solvent/Detergent Treatment", BIOLOGICALS., vol. 30, no. 3, 1 September 2002 (2002-09-01), GB, pages 197 - 205, XP055402680, ISSN: 1045-1056, DOI: 10.1006/biol.2002.0328
- VICTOR E BUCKWOLD ET AL: "Bovine viral diarrhea virus as a surrogate model of hepatitis C virus for the evaluation of antiviral agents", ANTIVIRAL RESEARCH, vol. 60, no. 1, 1 September 2003 (2003-09-01), NL, pages 1 - 15, XP055402683, ISSN: 0166-3542, DOI: 10.1016/S0166-3542(03)00174-8
- ANONYMOUS: "ICH Topic Q 5 A (R1) Quality of Biotechnological Products: Viral Safety Evaluation of Biotechnology Products Derived from Cell Lines of Human or Animal Origin", INTERNET CITATION, 1 October 1997 (1997-10-01), pages 1 - 29, XP003035110, Retrieved from the Internet <URL:http://www.ema.europa.eu/docs/en_GB/document_library/Scientific_guideline/2009/09/WC500002801.pdf> [retrieved on 20150301]
- CHANG JEN-TING ET AL: "Inactivation strategy for pseudorabies virus in milk for production of biopharmaceuticals", JAPANESE JOURNAL OF VETERINARY RESEARCH, 1 November 2010 (2010-11-01), pages 179 - 183, XP093185804, Retrieved from the Internet <URL:https://pubmed.ncbi.nlm.nih.gov/21180258/> DOI: 10.14943/jjvr.58.3-4.179

## Description

### PRIORITY CLAIM

This patent application claims priority to U. S. Provisional Patent Application Serial No. 61/423,512 filed December 15, 2010.

### FIELD

The present specification relates to methods of inactivating viral contaminants during the manufacturing of proteins.

### INTRODUCTION

The use of pharmaceutical compositions comprising a therapeutic protein has continued to increase in importance as a method of treating many diseases, disorders, or conditions that affect an individual's heath. Many proteins used in a pharmaceutical composition are typically obtained through recombinant production using a mammalian cell line or by purification from a biological fluid. However, a therapeutic protein manufactured by such methods may be contaminated with a contagious pathogenic virus deleterious to the health of an individual. Therefore, it is important to process a therapeutic protein to eliminate any contaminating viral activity, thereby ensuring that the resulting pharmaceutical composition is safe to administer to an individual.

There are currently many different methods for inactivating contagious pathogenic viruses, including, e.g., heat-inactivation, solvent/detergent (S/D) inactivation, pH inactivation, chemical inactivation, and/or ultraviolet irradiation inactivation. Of these, S/D inactivation is perhaps the most widely accepted virucidal method because nearly all of the significant human pathogens are enveloped viruses susceptible to membrane disruption by solvents and detergents. In addition, the S/D method better preserves the content and biological activity of the fluid being treated when compared to other virucidal methods. For example, inactivation methods using heat, acidic pH, chemicals and irradiation are problematic as these agents are harsh and/or invasive and tend to denature or otherwise inactivate the therapeutic protein being purified.

In the S/D inactivation method, an organic solvent and a detergent are mixed with a fluid including the protein being purified and incubated. The solvent creates an environment promoting aggregation between the detergent and the lipid membrane encapsulating the virus, and the detergent disrupts the interactions between molecules in this lipid membrane. Once disrupted, an enveloped virus can no longer bind to and infect a cell and is unable to reproduce because an intact lipid membrane is essential for such activities. This inactivation is generally performed at a temperature over 20°C because higher temperatures facilitate the disruption of the envelope. For example, typical conditions used in accordance with the World Health Organization (WHO) guidelines are 0.3% tri(n-butyl) phosphate (TNBP) and 1% polysorbate 80 sorbitan monooleate (TWEEN^{®} 80) incubated at 24°C for a minimum of 6 hours, or 0.3% TNBP and 1% polyoxyethylene octyl phenyl ether (TRITON^{®} X-100) incubated at 24°C for a minimum of 4 hours. See, e.g., WHO Technical Report, Annex 4 Guidelines on viral inactivation and removal procedures intended to assure the viral safety of human blood plasma products," Series No. 924, p 151-Roberts, P. L. (BIOLOGICALS, vol. 36, (5), 330-335, 2009) described virus 224, (2004). inactivation by solvent/detergent treatment using Triton X-100 in a high purity factor VIII preparation. Chang et al. (2010, Jpn J Vet Res 58(3-4): 179-183) describe an inactivation strategy for pseudorabies virus in milk for production of biopharmaceuticals.

Incubating the solvent/detergent mixture to a temperature over 20°C has several drawbacks. First, inactivation at higher temperatures is time consuming since pre-heating the mixture to temperatures above 20°C adds about two to about six hours to the overall processing time. Second, incubation at this higher temperature, in conjunction with the agitation necessary to facilitate uniform heating, enhances protein aggregate formation resulting in a reduction in the yield of active and/or useful protein. Exposure to temperatures above 20°C also increases a protein's susceptibility to degradation. The present specification discloses a novel S/D method for inactivating contagious pathogenic viruses from a sample that addresses these drawbacks. The method disclosed herein uses incubation times below two hours and incubation temperatures below 20°C. Decreasing the incubation time reduces the overall time necessary to process and/or manufacture a protein obtained through recombinant production or biological fluid purification. In addition, decreasing both the time and temperature result in improved protein yields by reducing its susceptibility to aggregation and degradation.

### SUMMARY OF THE TECHNICAL TEACHINGS

The present invention is defined by the appended claims. Also disclosed herein are the following aspects.

Thus, aspects of the present specification disclose methods of inactivating a lipid-coat containing virus, the methods comprising the steps of a) providing a fluid comprising a protein having an activity; b) mixing an organic solvent and a surfactant with the fluid, thereby creating a mixture; and c) incubating the mixture for no more than about 120 minutes; wherein both steps (b) and (c) are performed at a temperature of no higher than about 20°C; wherein the mixture after incubation is essentially free of a viable lipid-coat containing virus; and wherein the protein after incubation has an activity of at least 25% of the activity provided in step (a). The fluid can be a cell lysate, a cell supernatant, an elution from a previous purification step, or a biological fluid. The protein can be obtained through recombinant production using a cell line or by purification from a biological fluid. The organic solvent can be an ether, an alcohol, or an alkylphosphate like a dialkylphosphate or a trialkylphosphate. The surfactant can be an ionic surfactant like an anion surfactant or cationic surfactant, a zwitterionic (amphoteric) surfactant, or a non-ionic surfactant. The method may, or may not, further comprises a step of removing the solvent, surfactant and/or the non-viable virus from the mixture after step (c).

Other aspects of the present specification disclose a protein essentially free of a lipid-coat containing virus obtained from a method comprising the steps of: a) providing a fluid comprising a protein having an activity; b) mixing an organic solvent and a surfactant with the fluid, thereby creating a mixture; and c) incubating the mixture for no more than about 120 minutes; wherein both steps (b) and (c) are performed at a temperature of no higher than about 20°C; wherein the mixture after incubation is essentially free of a viable lipid-coat containing virus; and wherein the protein after incubation has an activity of at least 25% of the activity provided in step (a).

Yet other aspects of the present specification disclose methods of inactivating a lipid-coat containing virus, the methods comprising the steps of: a) providing a fluid comprising a Factor VIII having an activity; b) mixing an organic solvent and a surfactant with the fluid, thereby creating a mixture; and c) incubating the mixture for no more than about 120 minutes; wherein both steps (b) and (c) are performed at a temperature of no higher than about 20°C; wherein the mixture after incubation is essentially free of a viable lipid-coat containing virus; and wherein the Factor VIII after incubation has an activity of at least 25% of the activity provided in step (a).

Still other aspects of the present specification disclose a Factor VIII essentially free of a lipid-coat containing virus obtained from a method comprising the steps of: a) obtaining a fluid comprising a Factor VIII having an activity; b) mixing an organic solvent and a surfactant with the fluid, thereby creating a mixture; and c) incubating the mixture for no more than about 120 minutes; wherein both steps (b) and (c) are performed at a temperature of no higher than about 20°C; wherein the mixture after incubation is essentially free of a viable lipid-coat containing virus; and wherein the Factor VIII after incubation has an activity of at least 25% of the activity provided in step (a).

### DESCRIPTION

The technical disclosure set out below may in some respects go beyond the scope of the invention, which is defined by the appended claims. Elements and aspects of the disclosure which do not fall within the scope of the claims are provided for information only, namely to place the actual invention claimed in a broader technical context.

Aspects of the present specification disclose, in part, a lipid-coat containing virus. A complete virus particle, known as a virion, consists of nucleic acid, either DNA or RNA, surrounded by a protective coat of protein called a capsid. Viruses can be grouped into non-enveloped and enveloped viruses. As used herein, the term "lipid-coat containing virus" refers to any virus comprising a membrane or envelope including lipid, such as, e.g., an envelope virus. Enveloped viruses have their capsid enclosed by a lipoprotein membrane, or envelope. This envelope is derived from the host cell as the virus "buds" from its surface and consists mostly of lipids not encoded by the viral genome. Even though it carries molecular determinants for attachment and entry into target cells, and is essential for the infectivity of enveloped viruses, it is not subject to drug resistance or antigenic shift. Enveloped viruses range in size from about 45-55 nm to about 120-200 nm. Lipid-coat containing viruses which can infect mammalian cells include DNA viruses like a herpesviridae virus, a poxviridae virus, or a hepadnaviridae virus; RNA viruses like a flaviviridae virus, a togaviridae virus, a coronaviridae virus, a deltavirus virus, an orthomyxoviridae virus, a paramyxoviridae virus, a rhabdoviridae virus, a bunyaviridae virus, or a filoviridae virus; and reverse transcribing viruses like a retroviridae virus or a hepadnaviridae virus. Non-limiting examples of lipid-coat containing viruses include a human immunodeficiency virus, a sindbis virus, a herpes simplex virus, a pseudorabies virus, a sendai virus, a vesicular stomatitis virus, a West Nile virus, a bovine viral diarrhea virus, a corona virus, an equine arthritis virus, a severe acute respiratory syndrome virus, Moloney murine leukemia virus, or a vaccinia virus.

A non-enveloped virus refers to a virus whose capsid lacks a lipoprotein membrane, or envelope. In a non-enveloped virus, the capsid mediates attachment to and penetration into host cells. Capsids are generally either helical or icosahedral. Non-enveloped viruses range in size from about 18-26 nm to about 70-90 nm. Non-enveloped viruses which can infect mammalian cells include, e.g., a parvoviridae virus, an adenoviridae virus, a birnaviridae virus, a papillomaviridae virus, a polyomaviridae virus, a picornaviridae virus, a reoviridae virus, and a calciviridae virus.
The present invention relates to inactivating a pseudorabies virus as defined by the appended claims.

Aspects of the present specification disclose, in part, a fluid comprising a protein having an activity. A fluid may be any fluid having the possibility of being contaminated by a virus. Non-limiting examples of a fluid include, e.g., any purified, partially purified or crude liquid or colloid including, e.g., an elution from a previous purification step; tissue and cell culture extract like a cell lysate, a cell supernatant, placental extracts, or an ascites fluid; a biological fluid like blood, plasma, serum, milk, saliva, semen; or any other fluid that includes a protein having an activity.

A protein having an activity may be any protein of interest in which elimination of any contaminating viral activity is desired. A protein disclosed herein can be a blood protein such as, e.g., a blood coagulation protein, albumin, and/or an immunoglobulin. Non-limiting examples of a blood protein include ADAMTS-13, α1-antiplasmin, α2-antiplasmin, antithrombin, antithrombin III, cancer procoagulant, erythropoietin, Factor II, Factor V, Factor VI, Factor VII, Factor VIII, Factor IX, Factor X, Factor XI, Factor XII, Factor XIII, fibronectin, fibrinogen, heparin cofactor II, high-molecular-weight kininogen, intramuscular immunoglobulin, intravenous immunoglobulin, plasminogen, plasminogen activator inhibitor-1, plasminogen activator inhibitor-2, prekallikrein, protein C, protein S, protein Z, protein Z-related protease inhibitor, tissue factor, tissue plasminogen activator, urokinase, or Von Willebrand Factor.
The protein in accordance with the present invention as defined by the appended claims is Factor VIII.

A protein disclosed herein may be obtained from an organism that naturally expresses the protein, from a transgenic organism genetically-engineered to express the protein, or from a cell line recombinantly producing the protein. Non-limiting examples of an organism include birds and mammals, such as, e.g., mice, rats, goats, sheep, horses, donkeys, cows, primates and humans. Non-limiting examples of a transgenic organisms include organisms disclosed herein that have been genetically-engineered to express a protein of interest. A protein disclosed herein from an organism or transgenic organism may be obtained from a biological fluid, tissue or organ extract, or other source from an organism using routine methods known in the art. For example, to obtain a blood protein, whole blood from an organism or transgenic organism can be drawn in a serum separator tube. The blood is allowed to clot and the clotted blood centrifuged to pellet the debris. The resulting serum sample (i.e., the supernatant) can then be aliquoted and/or stored at -20°C until needed. Non-limiting examples of specific protocols for blood collection and serum preparation are described in, e.g., Di Lorenzo and Strasinger, BLOOD COLLECTION IN HEALTHCARE (F.A. Davis Company, 2001); and Diana Garza & Kathleen Becan-McBride, PHLEBOTOMY HANDBOOK: BLOOD COLLECTION ESSENTIALS (Prentice Hall, 6th ed., 2002).

Alternatively, various prokaryote and/or eukaryotic expression systems may also be employed to recombinantly express a protein disclosed herein. Expression systems can include any of a variety of characteristics including, without limitation, inducible expression, non-inducible expression, constitutive expression, tissue-specific expression, cell-specific expression, viral-mediated expression, stablyintegrated expression, and transient expression. How to make and use such expression systems are known in the art.

Generally, a polynucleotide encoding the protein of interest is cloned into an expression vector. Prokaryote expression vectors typically comprise an origin of replication, a suitable promoter and/or enhancer elements, and also sites necessary ribosome binding, polyadenylation, transcriptional termination, as well as 5' flanking non-transcribed sequences and other non-transcribed genetic elements. Exemplary prokaryotic vectors include pET and pRSET using promoters such as, e.g., a bacteriophage T7 promoter. Eukaryotic expression vectors typically comprise an origin of replication, a suitable promoter and/or enhancer elements, and also sites necessary ribosome binding, polyadenylation, splicing, transcriptional termination, as well as 5' flanking non-transcribed sequences and other non-transcribed genetic elements. Exemplary yeast vectors include pAO, pMET, pPIC, pPICZ, and pYES using promoters such as, e.g., AOX1, AUG1, GAP, and GAL1. Exemplary insect vectors include pAc5, pBAC, pIB, pMIB, pMT using promoters such as, e.g., PH, p10, MT, Ac5, OpIE2, gp64, and polh. Exemplary mammalian vectors include pBPV, pCMV, pCMVTNT, pDNA, pDisplay, pMSG, pOG44, pQBI25, pRc/RSV, pSECTag, pSECTag2, pSG, pSV2cat, pSVK3, pSVL, pUCIG-MET, pVAX1, pWLneo, and pXT1 using promoters such as, e.g., beta-casein, beta-lactoglobulin, whey acid promoter, HSV thymidine kinase, early and late simian virus 40 (SV40), LTRs from retrovirus, and mouse metallothionein-1. Selectable markers include Ampicillin, Chloramphenicol transferase, Kanamycin, Neomycin, and Tetracycline. Suitable expression vectors are known in the art and commercially available.

Cell capable of expressing a compatible vector include prokaryotic cells, eukaryotic cells, and cell lines derived from prokaryotic and eukaryotic cells. Non-limiting examples of prokaryotic strains include those derived from, *e.g., Escherichia coli, Bacillus subtilis, Bacillus licheniformis, Bacteroides fragilis, Clostridia perfringens, Clostridia difficle, Caulobacter crescentus, Lactococcus lactis, Methylobacterium extorquens, Neisseria meningirulls, Neisseria meningitidis, Pseudomonas fluorescens* and *Salmonella typhimurium.* Non-limiting examples of yeast strains include those derived from, *e.g., Pichia pastoris, Pichia methanolica, Pichia angusta, Schizosaccharomyces pombe, Saccharomyces cerevisiae* and *Yarrowia lipolytica.* Plant cells and cell lines derived from plants include cells from, e.g., species of monocots, such as, *e.g., Zea mays* and species of dicots, such as, *e.g., Arabidopsis thaliana, Triticum aestivum, Lemna gibba* and *Lemna minor.* Insect cells and cell lines derived from insects include cells from, *e.g., Spodoptera frugiperda, Trichoplusia ni, Drosophila melanogaster* and *Manduca sexta.* Non-limiting examples of insect cell lines include High-Five, K_{C}, Schneider's Drosophila line 2 (S2), SF9, and SF21 cell lines. Mammalian cells and cell lines derived from mammalian cells include cells from, e.g., mouse, rat, hamster, porcine, bovine, equine, primate and human. Non-limiting examples of mammalian cell lines include 1A3, 3T3, 6E6, 10T1/2, APRT, BALB/3T3, BE (2)-C, BHK, BT, C6, C127, CHO, CHP3, COS-1, COS-7, CPAE, ESK-4, FB2, GH1, GH3, HeLa, HEK-293, HepG2, HL-60, IMR-32, L2, LLC-PK1, L-M, MCF-7, NB4, NBL-6, NCTC, Neuro 2A, NIE-115, NG108-15, NIH3T3, PC12, PK15, SBAC, SH-SY5Y, SK-Hep, SK-N-DZ, SK-N-F1, SK-N-SH, ST, SW-13, and VV-1 cell lines. Cell lines may be obtained from the American Type Culture Collection, European Collection of Cell Cultures and/or the German Collection of Microorganisms and Cell Cultures.

After initial harvesting from an organism, transgenic organism, or cell culture system, a protein disclosed herein may undergo a protein purification process. Generally, protein purification include capture of the protein to a more concentrated form, intermediate purification steps to remove impurities, polishing to remove additional impurities and protein variants. See, e.g., Current Protocols in Protein Science, "Conventional chromatographic Separations," Ch. 8-9, (John Wiley & Sons Inc., Hoboken, N.J., 1995). Common methods of capture include affinity chromatography, gel filtration, precipitation, and/or size exclusion chromatography. Processes useful as intermediate or polishing steps include cation-exchange chromatography, anion-exchange chromatography, hydrophobic-interaction chromatography, and ceramic hydroxyapatite chromatography, reverse-phase HPLC, gel filtration, precipitation, diafiltration, affinity chromatography, or chromatofocusing.

The method disclosed herein may be performed at any point during this purification process. In general, greater reagent quantities and longer incubation times are necessary when the method disclosed herein is performed at the beginning of a manufacturing process rather than later in the process. This is because process volumes are greater and the product is typically less pure at the beginning, whereas later on impurities have been reduced and the product is in most cases better defined and of greater purity and potency with reduced volume, or where viral load has been reduced or diminished by previous purification procedures.

Aspects of the present specification provide, in part, mixing a fluid disclosed herein with an organic solvent and a surfactant, thereby creating a mixture. Mixing of the fluid with the organic solvent and surfactant may be for any length of time, with the proviso that the mixing time used results in a mixture that sufficiently incorporates the organic solvent and a surfactant with the fluid. As such, the mixing should ensure that 1) the organic solvent promotes contact between a surfactant and the lipoprotein envelope encapsulating the lipid-coat containing virus and 2) the surfactant disrupts the interactions between molecules in the lipoprotein membrane of a lipid-coat containing virus. In aspects thereof mixing of an organic solvent and surfactant is for a time of, e.g., no more than 1 minute, no more than 5 minutes, or no more than 10 minutes. In other aspects thereof, mixing of an organic solvent and surfactant is for a time of between, e.g., about 1 minute to about 5 minutes, about 2 minutes to about 5 minutes, about 3 minutes to about 5 minutes, about 1 minute to about 10 minutes, about 2 minutes to about 10 minutes, about 3 minutes to about 10 minutes, about 4 minutes to about 10 minutes, or about 5 minutes to about 10 minutes. As disclosed herein, mixing of an organic solvent and a surfactant is performed at a temperature of no higher than about 20°C.

Either a single organic solvent may be mixed with the fluid disclosed herein, or a plurality of organic solvents may be mixed with the fluid disclosed herein. In aspect thereof, a fluid may be mixed with at least one, at least two, at least three, at least four, or at least five organic solvents. Useful organic solvents create an environment promoting contact between a surfactant and the lipoprotein envelope encapsulating the virus. As such, any organic solvent that promotes such contact may be used in the method disclosed herein, including, without limitation, an ether, an alcohol, an alkylphosphate like a dialkylphosphate or a trialkylphosphate, or any combination thereof.

Ethers useful in the method disclosed herein include those having the formula R¹-O-R², wherein, R¹ and R² are independently C₁-C₁₈ alkyl or C₁-C₁₈ alkenyl which can contain an oxygen or sulfur atom, preferably C₁-C₁₈ alkyl or C₁-C₁₈ alkenyl. Non-limiting examples of ethers include dimethyl ether, diethyl ether, ethyl propyl ether, methyl-butyl ether, methyl isopropyl ether and methyl isobutyl ether. Alcohols useful in the method disclosed herein include those having C₁-C₈ alkyl groups or C₁-C₈ alkenyl groups. Non-limiting examples of alcohols include methanol, ethanol, propanol, isopropanol, n-butanol, isobutanol, n-pentanol and the isopentanols. Alkylphosphates useful in the method disclosed herein include those having C₁-C₁₈ alkyl groups or C₁-C₁₈ alkenyl groups, either of which may contain an oxygen or sulfur atom. Non-limiting examples of alkylphosphates include dialkylphosphates like di-(n-butyl)phosphate, di-(t-butyl)phosphate, di-(n-hexyl)phosphate, di-(2-ethylhexyl)phosphate, di-(n-decyl)phosphate, or ethyl di(n-butyl) phosphate; and trialkylphosphates like tri-(n-butyl)phosphate, tri-(t-butyl)phosphate, tri-(n-hexyl)phosphate, tri-(2-ethylhexyl)phosphate, or tri-(n-decyl)phosphate. Other non-limiting examples of organic solvent useful in the methods disclosed herein can be found in, e.g., Winslow, et al., Methods and Compositions for Simultaneously Isolating Hemoglobin from Red Blood Cells and Inactivating Viruses, U.S. 2008/0138790.
In the present invention as defined by the appended claims, tri-(n-butyl)phosphate is used.

Any concentration of organic solvent may be used, with the proviso that the concentration used is sufficient to promote aggregation between the surfactant and the lipoprotein membrane of a lipid-coat containing virus. In aspects thereof, the surfactant is used at a final concentration of, e.g., about 0.01% (v/v), about 0.05% (v/v), about 0.075% (v/v), about 0.1% (v/v), about 0.2% (v/v), about 0.3% (v/v), about 0.4% (v/v), about 0.5% (v/v), about 0.6% (v/v), about 0.7% (v/v), about 0.8% (v/v), about 0.9% (v/v), or about 1.0% (v/v). In other aspects thereof, the surfactant is used at a final concentration of, e.g., at least 0.01% (v/v), at least 0.05% (v/v), at least 0.075% (v/v), at least 0.1% (v/v), at least 0.25% (v/v), at least 0.5% (v/v), at least 0.75% (v/v), or at least 1.0% (v/v). In yet other aspects thereof, the surfactant is used at a final concentration of, e.g., about 0.1% (v/v) to about 0.5% (v/v), about 0.1% (v/v) to about 0.75% (v/v), about 0.1% (v/v) to about 1.0% (v/v), about 0.2% (v/v) to about 0.5% (v/v), about 0.2% (v/v) to about 0.75% (v/v), about 0.2% (v/v) to about 1.0% (v/v), about 0.5% (v/v) to about 0.75% (v/v), or about 0.5% (v/v) to about 1.0% (v/v). In the present invention as defined by the appended claims, the final concentration of tri(n-butyl)phosphate is 0.21% (v/v).

Surfactants are compounds that lower the surface tension of a liquid, allowing easier spreading, and lowering of the interfacial tension between two liquids, or between a liquid and a solid. Either a single surfactant may be mixed with the fluid disclosed herein, or a plurality of surfactants may be mixed with the fluid disclosed herein. In aspect of thereof, a fluid may be mixed with at least one, at least two, at least three, at least four, or at least five surfactants. Useful surfactants disrupt the interactions between molecules in the lipoprotein membrane of a lipid-coat containing virus. As such, any surfactant that facilitates such disruption may be used in the method disclosed herein, including, without limitation, ionic surfactants, zwitterionic (amphoteric) surfactants, non-ionic surfactants, or any combination therein.

Ionic surfactants include anionic surfactants based on permanent (sulfate, sulfonate, phosphate) or pH dependent (carboxylate) anions. Anionic surfactants include, without limitation, alkyl sulfates like ammonium lauryl sulfate and sodium lauryl sulfate (SDS); alkyl ether sulfates like sodium laureth sulfate and sodium myreth sulfate; docusates like dioctyl sodium sulfosuccinate; sulfonate fluorosurfactants like perfluorooctanesulfonate (PFOS) and perfluorobutanesulfonate; alkyl benzene sulfonates; alkyl aryl ether phosphates; alkyl ether phosphates; alkyl carboxylates like fatty acid salts and sodium stearate; sodium lauroyl sarcosinate; and carboxylate fluorosurfactants like perfluorononanoate and perfluorooctanoate.

Ionic surfactants also include cationic surfactants based on permanent or pH dependent cations. Cationic surfactants include, without limitation, alkyltrimethylammonium salts like cetyl trimethylammonium bromide (CTAB) and cetyl trimethylammonium chloride (CTAC); cetylpyridinium chloride (CPC); polyethoxylated tallow amine (POEA); benzalkonium chloride (BAC); benzethonium chloride (BZT); 5-Bromo-5-nitro-1,3-dioxane; dimethyldioctadecylammonium chloride; and dioctadecyldimethylammonium bromide (DODAB), as well as pH-dependent primary, secondary or tertiary amines like surfactants where the primary amines become positively charged at pH < 10, or the secondary amines become charged at pH < 4, like octenidine dihydrochloride.

Zwitterionic surfactants are based on primary, secondary or tertiary amines or quaternary ammonium cation with a sulfonate, a carboxylate, or a phosphate. Zwitterionic surfactants include, without limitation, 3-[(3-Cholamidopropyl)dimethylammonio]-1-propanesulfonate (CHAPS); sultaines like cocamidopropyl hydroxysultaine; betaines like cocamidopropyl betaine; or lecithins.

Non-ionic surfactants are less denaturing and as such are useful to solubilize membrane proteins and lipids while retaining protein-protein interactions. Non-limiting examples of surfactants include polyoxyethylene glycol sorbitan alkyl esters like polysorbate 20 sorbitan monooleate (TWEEN^{®} 20), polysorbate 40 sorbitan monooleate (TWEEN^{®} 40), polysorbate 60 sorbitan monooleate (TWEEN^{®} 60), polysorbate 61 sorbitan monooleate (TWEEN^{®} 61), polysorbate 65 sorbitan monooleate (TWEEN^{®} 65), polysorbate 80 sorbitan monooleate (TWEEN^{®} 80), and polysorbate 81 sorbitan monooleate (TWEEN^{®} 81); poloxamers (polyethylene-polypropylene copolymers), like Poloxamer 124 (PLURONIC^{®} L44), Poloxamer 181 (PLURONIC^{®} L61), Poloxamer 182 (PLURONIC^{®} L62), Poloxamer 184 (PLURONIC^{®} L64), Poloxamer 188 (PLURONIC^{®} F68), Poloxamer 237 (PLURONIC^{®} F87), Poloxamer 338 (PLURONIC^{®} L108), Poloxamer 407 (PLURONIC^{®} F127); alkyl phenol polyglycol ethers; polyethylene glycol alkyl aryl ethers; polyoxyethylene glycol alkyl ethers, like octaethylene glycol monododecyl ether, pentaethylene glycol monododecyl ether, BRIJ^{®} 30, and BRIJ^{®} 35; 2-dodecoxyethanol (LUBROL^{®}-PX); polyoxyethylene glycol octylphenol ethers like polyoxyethylene (4-5) p-t-octyl phenol (TRITON^{®} X-45) and polyoxyethylene octyl phenyl ether (TRITON^{®} X-100); polyoxyethylene glycol alkylphenol ethers like Nonoxynol-9; phenoxypolyethoxylethanols like nonyl phenoxypolyethoxylethanol and octyl phenoxypolyethoxylethanol; glucoside alkyl ethers like octyl glucopyranoside; maltoside alkyl ethers like dodecyl maltopyranoside; thioglucoside alkyl ethers like heptyl thioglucopyranoside; digitonins; glycerol alkyl esters like glyceryl laurate; alkyl aryl polyether sulfates; alcohol sulfonates; sorbitan alkyl esters; cocamide ethanolamines like cocamide monoethanolamine and cocamide diethanolamine; sucrose monolaurate; dodecyl dimethylamine oxide, and sodium cholate. Other non-limiting examples of surfactants useful in the methods disclosed herein can be found in, e.g., Winslow, et al., Methods and Compositions for Simultaneously Isolating Hemoglobin from Red Blood Cells and Inactivating Viruses, U.S. 2008/0138790; Pharmaceutical Dosage Forms and Drug Delivery Systems (Howard C. Ansel et al., eds., Lippincott Williams & Wilkins Publishers, 7th ed. 1999); Remington: The Science and Practice of Pharmacy (Alfonso R. Gennaro ed., Lippincott, Williams & Wilkins, 20th ed. 2000); Goodman & Gilman's The Pharmacological Basis of Therapeutics (Joel G. Hardman et al., eds., McGraw-Hill Professional, 10th ed. 2001); and Handbook of Pharmaceutical Excipients (Raymond C. Rowe et al., APhA Publications, 4th edition 2003). In the present invention as defined by the appended claims, polyoxyethylene octyl phenyl ether and polysorbate 80 sorbitan monooleate are used.

Any concentration of surfactant may be used, with the proviso that the concentration used is sufficient to disrupt the interactions between molecules in the lipoprotein membrane of a lipid-coat containing virus causing inactivation of the virus. In aspects of thereof, the surfactant is used at a concentration of, e.g., about 0.01% (v/v), about 0.05% (v/v), about 0.075% (v/v), about 0.1% (v/v), about 0.2% (v/v), about 0.3% (v/v), about 0.4% (v/v), about 0.5% (v/v), about 0.6% (v/v), about 0.7% (v/v), about 0.8% (v/v), about 0.9% (v/v), about 1.0% (v/v), about 2.0% (v/v), about 3.0% (v/v), about 4.0% (v/v), about 5.0% (v/v), about 6.0% (v/v), about 7.0% (v/v), about 8.0% (v/v), about 9.0% (v/v), or about 10.0% (v/v). In other aspects thereof, the surfactant is used at a concentration of, e.g., at least 0.01% (v/v), at least 0.05% (v/v), at least 0.075% (v/v), at least 0.1% (v/v), at least 0.25% (v/v), at least 0.5% (v/v), at least 0.75% (v/v), at least 1.0% (v/v), at least 2.5% (v/v), at least 5.0% (v/v), at least 7.5% (v/v), or at least 10.0% (v/v). In yet other aspects thereof, the surfactant is used at a concentration of, e.g., about 0.1% (v/v) to about 0.5% (v/v), about 0.1% (v/v) to about 1.0% (v/v), about 0.2% (v/v) to about 0.5% (v/v), about 0.2% (v/v) to about 1.0% (v/v), about 0.2% (v/v) to about 2.0% (v/v),about 0.5% (v/v) to about 1.0% (v/v), about 0.5% (v/v) to about 5.0% (v/v), or about 1.0% (v/v) to about 10.0% (v/v).
In the present invention as defined by the appended claims, the final concentration of polyoxyethylene octyl phenyl ether is 0.7% (v/v), and the final concentration of polysorbate 80 sorbitan monooleate is 0.21% (v/v).

A fluid disclosed herein may be mixed with a single organic solvent and a plurality of surfactants, a plurality of organic solvents and a single of surfactant, or a plurality of organic solvents and a plurality of surfactants. Typically, the final concentrations of an organic solvent and a single surfactant in a fluid is about 0.1% (v/v) to about 5% (v/v) of organic solvent and about 0.1% (v/v) to about 10% (v/v) of surfactant. When a plurality of surfactants are mixed with a fluid, the final concentration of an organic solvent is about 0.1% (v/v) to about 5% (v/v), the final concentration of one surfactant is about 0.1% (v/v) to about 10% (v/v), about 0.5% (v/v) to about 5% (v/v), or about 0.5% (v/v) to about 1.0% (v/v), and the final concentration of the remainder of surfactants is about 0.1% (v/v) to about 5% (v/v), about 0.1% (v/v) to about 1.0% (v/v), or about 0.2% (v/v) to about 4% (v/v).

In an aspect thereof, a fluid is mixed with tri(n-butyl) phosphate and polyoxyethylene octyl phenyl ether (TRITON^{®} X-100) such that the final concentration is from, e.g., about 0.1% (v/v) to about 1.0% (v/v)tri(n-butyl) phosphate and about 1.0% (v/v) to about 10.0% (v/v) polyoxyethylene octyl phenyl ether (TRITON^{®} X-100). In another aspect of thereof, a fluid is mixed with tri(n-butyl) phosphate and polysorbate 80 sorbitan monooleate (TWEEN^{®} 80) such that the final concentration is from, e.g., about 0.1% (v/v) to about 1.0% (v/v)tri(n-butyl) phosphate and about 1.0% (v/v) to about 10.0% (v/v) polysorbate 80 sorbitan monooleate (TWEEN^{®} 80).

In another aspect of thereof, a fluid is mixed with tri(n-butyl) phosphate, polyoxyethylene octyl phenyl ether (TRITON^{®} X-100), and polysorbate 80 sorbitan monooleate (TWEEN^{®} 80) such that the final concentration is from, e.g., about 0.1% (v/v) to about 5.0% (v/v) tri(n-butyl) phosphate, about 0.5% (v/v) to about 10.0% (v/v) polyoxyethylene octyl phenyl ether (TRITON^{®} X-100), and about 0.1% (v/v) to about 5.0% (v/v) polysorbate 80 sorbitan monooleate (TWEEN^{®} 80). In yet another aspect thereof, a fluid is mixed with tri(n-butyl) phosphate, polyoxyethylene octyl phenyl ether (TRITON^{®} X-100), and polysorbate 80 sorbitan monooleate (TWEEN^{®} 80) such that the final concentration is from, e.g., about 0.1% (v/v) to about.0.7% (v/v)tri(n-butyl) phosphate, about 0.6% (v/v) to about 1.4% (v/v) polyoxyethylene octyl phenyl ether (TRITON^{®} X-100), and about 0.1% (v/v) to about 0.7% (v/v) polysorbate 80 sorbitan monooleate (TWEEN^{®} 80). In still another aspect thereof, a fluid is mixed with tri(n-butyl) phosphate, polyoxyethylene octyl phenyl ether (TRITON^{®} X-100), and polysorbate 80 sorbitan monooleate (TWEEN^{®} 80) such that the final concentration is from, e.g., about 0.1% (v/v) to about.0.5% (v/v)tri(n-butyl) phosphate, about 0.7% (v/v) to about 1.3% (v/v) polyoxyethylene octyl phenyl ether (TRITON^{®} X-100), and about 0.1% (v/v) to about 0.5% (v/v) polysorbate 80 sorbitan monooleate (TWEEN^{®} 80).

In another aspect thereof, a fluid is mixed with tri(n-butyl) phosphate, polyoxyethylene octyl phenyl ether (TRITON^{®} X-100), and polysorbate 80 sorbitan monooleate (TWEEN^{®} 80) such that the final concentration is from, e.g., about 0.2% (v/v) to about 0.5% (v/v)tri(n-butyl) phosphate, about 0.7% (v/v) to about 1.3% (v/v) polyoxyethylene octyl phenyl ether (TRITON^{®} X-100), and about 0.2% (v/v) to about 0.5% (v/v) polysorbate 80 sorbitan monooleate (TWEEN^{®} 80). In yet another aspect thereof, a fluid is mixed with tri(n-butyl) phosphate, polyoxyethylene octyl phenyl ether (TRITON^{®} X-100), and polysorbate 80 sorbitan monooleate (TWEEN^{®} 80) such that the final concentration is from, e.g., about 0.2% (v/v) to about 0.4% (v/v)tri(n-butyl) phosphate, about 0.8% (v/v) to about 1.2% (v/v) polyoxyethylene octyl phenyl ether (TRITON^{®} X-100), and about 0.2% (v/v) to about 0.4% (v/v) polysorbate 80 sorbitan monooleate (TWEEN^{®} 80). In still another aspect thereof, a fluid is mixed with tri(n-butyl) phosphate, polyoxyethylene octyl phenyl ether (TRITON^{®} X-100), and polysorbate 80 sorbitan monooleate (TWEEN^{®} 80) such that the final concentration is from, e.g., about 0.3% (v/v) (v/v)tri(n-butyl) phosphate, about 1.0% (v/v) polyoxyethylene octyl phenyl ether (TRITON^{®} X-100), and about 0.3% (v/v) polysorbate 80 sorbitan monooleate (TWEEN^{®} 80).

In the present invention as defined by the appended claims, a fluid is mixed with tri-(n-butyl)phosphate, polyoxyethylene octyl phenyl ether, and polysorbate 80 sorbitan monooleate, thereby creating a mixture, wherein the final concentration of tri(n-butyl) phosphate is 0.21% (v/v), the final concentration of polyoxyethylene octyl phenyl ether is 0.7% (v/v), and the final concentration of polysorbate 80 sorbitan monooleate is 0.21% (v/v).

After mixing the fluid with of an organic solvent and surfactant, the resulting mixture is incubated for a specified time at a specified temperature. Any incubation time may be used, with the proviso that the time used results in a mixture after incubation that is essentially free of a viable lipid-coat containing virus. In aspects thereof, a fluid is incubated for a time of, e.g., about 10 minutes, about 20 minutes, about 30 minutes, about 40 minutes, about 50 minutes, about 60 minutes, about 70 minutes, about 80 minutes, about 90 minutes, about 100 minutes, about 110 minutes, or about 120 minutes. In other aspects thereof, a fluid is incubated for a time of, e.g., no more than about 10 minutes, no more than about 20 minutes, no more than about 30 minutes, no more than about 40 minutes, no more than about 50 minutes, no more than about 60 minutes, no more than about 70 minutes, no more than about 80 minutes, no more than about 90 minutes, no more than about 100 minutes, no more than about 110 minutes, or no more than about 120 minutes. In yet other aspects thereof, a fluid is incubated for a time of between, e.g., about 10 minutes to about 60 minutes, about 10 minutes to about 90 minutes, about 10 minutes to about 120 minutes, about 30 minutes to about 60 minutes, about 30 minutes to about 90 minutes, about 30 minutes to about 120 minutes, about 60 minutes to about 90 minutes, about 60 minutes to about 120 minutes, or about 90 minutes to about 120 minutes.
In the present invention as defined by the appended claims, the mixture is incubated for no more than 120 minutes.

Aspects of the present specification provide, in part, wherein a fluid is mixed with an organic solvent and surfactant at a temperature of no higher than about 20°C, and wherein the mixture is incubated at a temperature of no higher than about 20°C. Any incubation temperature may be used, with the proviso that the temperature used is a temperature of no higher than about 20°C and results in a mixture after incubation that is essentially free of a viable lipid-coat containing virus and a protein with activity is recovered after incubation. Although typically incubated at the same temperature, a fluid may be mixed with an organic solvent and surfactant at one temperature, and the mixture incubated at a different temperature.

In aspects thereof, a fluid is mixed and the mixture is incubated at a temperature of, e.g., about 2°C, about 4°C, about 6°C, about 8°C, about 10°C, about 12°C, about 14°C, about 16°C, about 18°C, or about 20°C. In other aspects thereof, a fluid is mixed and incubated at a temperature of, e.g., no more than 2°C, no more than 4°C, no more than 6°C, no more than 8°C, no more than 10°C, no more than 12°C, no more than 14°C, no more than 16°C, no more than 18°C, or no more than 20°C. In yet other aspects thereof, a fluid is mixed and the mixture is incubated at a temperature of between, e.g., about 0°C to about 2°C, about 0°C to about 4°C, about 0°C to about 6°C, about 0°C to about 8°C, about 0°C to about 10°C, about 0°C to about 12°C, about 0°C to about 14°C, about 0°C to about 16°C, about 0°C to about 18°C, about 0°C to about 20°C, about 2°C to about 4°C, about 2°C to about 6°C, about 2°C to about 8°C, about 2°C to about 10°C, about 2°C to about 12°C, about 2°C to about 14°C, about 2°C to about 16°C, about 2°C to about 18°C, about 2°C to about 20°C, about 4°C to about 6°C, about 4°C to about 8°C, about 4°C to about 10°C, about 4°C to about 12°C, about 4°C to about 14°C, about 4°C to about 16°C, about 4°C to about 18°C, or about 4°C to about 20°C.

In one aspect, a mixture disclosed herein may be incubated for any length of time and at any temperature no higher than about 20°C with the proviso that the incubation time and temperature used results in a mixture after incubation that is essentially free of a viable lipid-coat containing virus and comprises a protein with activity. In aspects thereof, a mixture is incubated for no more than 120 minutes at a temperature of no higher than about 20°C, such as, e.g., no more than 90 minutes at a temperature of no higher than about 20°C, no more than 60 minutes at a temperature of no higher than about 20°C, or no more than 30 minutes at a temperature of no higher than about 20°C. In other aspects thereof, a mixture is incubated for no more than 120 minutes at a temperature of no higher than about 16°C, no more than 120 minutes at a temperature of no higher than about 12°C, or no more than 120 minutes at a temperature of no higher than about 8°C. In other aspects thereof, a mixture is incubated for about 10 minutes to about 120 minutes at a temperature of about 0°C to about 20°C, about 30 minutes to about 120 minutes at a temperature of about 0°C to about 16°C, about 30 minutes to about 120 minutes at a temperature of about 0°C to about 12°C, or about 30 minutes to about 120 minutes at a temperature of about 2°C to about 8°C. In yet other aspects thereof, a mixture is incubated for about 45 minutes to about 75 minutes at a temperature of about 2°C to about 8°C, about 60 minutes at a temperature of about 2°C to about 8°C, about 45 minutes to about 75 minutes at a temperature of about 4°C, or about 60 minutes at a temperature of about 4°C.
In the present invention as defined by the appended claims, the mixing and incubating are performed at a temperature of between 2°C to 8°C, and the mixture is incubated for no more than 120 minutes.

Aspects of the present specification provide, in part, a mixture after incubation that is essentially free of a viable lipid-coat containing virus. As used herein, the term "essentially free of a lipid-coat containing virus" means that only trace amounts of a viable lipid-coat containing virus can be detected or confirmed by the instrument or process being used to detect or confirm the presence or activity of the viable lipid-coat containing virus and that such trace amount of the viable lipid-coat containing virus is insufficient to be deleterious to the health of the human being. In an aspect thereof, a mixture after incubation is entirely free of a lipid-coat containing virus. As used herein, the term "entirely free of a lipid-coat containing virus" means that the presence of viable lipid-coat containing virus cannot be detected or confirmed within the detection range of the instrument or process being used to detect or confirm the presence or activity of the viable lipid-coat containing virus. A protein contained within a fluid that is essentially free or entirely free of a viable lipid-coat containing virus can be used to make a pharmaceutical composition that is safe to administer to a human being because the virus is insufficient to be deleterious to the health of the human being.
In the present invention as defined by the appended claims, the mixture after incubation is essentially free of a viable pseudorabies virus.

In another aspect, a mixture after incubation comprises less than 1 x 10¹ PFU/mL of a viable lipid-coat containing virus. A plaque-forming unit (PFU) is a measure of the number of virus particles capable of forming plaques per unit volume. It is a functional measurement rather than a measurement of the absolute quantity of particles since viral particles that are inactive, defective, or otherwise fail to infect their target cell will not produce a plaque and thus will not be counted. In other aspects thereof, a mixture after incubation comprises, e.g. less than 1 x 10⁰ PFU/mL of a viable lipid-coat containing virus, less than 1 x 10⁻¹ PFU/mL of a viable lipid-coat containing virus, 1 x 10⁻² PFU/mL of a viable lipid-coat containing virus, or 1 x 10⁻³ PFU/mL of a viable lipid-coat containing virus.

In yet another aspect thereof, a mixture after incubation comprises less than an ID₅₀ for a viable lipid-coat containing virus before the incubation. An ID₅₀ is the amount of virus that will infect 50% of a target population of cells. It is a functional measurement because viral particles that are inactive or otherwise defective will not infect their target cell and thus will not be counted. In other aspects thereof, a mixture after incubation comprises e.g., at least 10-fold less than the ID₅₀ for a viable lipid-coat containing virus, at least 100-fold less than the ID₅₀ for a viable lipid-coat containing virus, at least 200-fold less than the ID₅₀ for a viable lipid-coat containing virus, at least 300-fold less than the ID₅₀ for a viable lipid-coat containing virus, at least 400-fold less than the ID₅₀ for a viable lipid-coat containing virus, at least 500-fold less than the ID₅₀ for a viable lipid-coat containing virus, at least 600-fold less than the ID₅₀ for a viable lipid-coat containing virus, at least 700-fold less than the ID₅₀ for a viable lipid-coat containing virus, at least 800-fold less than the ID₅₀ for a viable lipid-coat containing virus, at least 900-fold less than the ID₅₀ for a viable lipid-coat containing virus, or at least 1000-fold less than the ID₅₀ for a viable lipid-coat containing virus before the incubation.

As used herein, the term "inactivating virus" or "virus inactivation" refers to a process where a virus can no longer infect cells, replicate, and propagate, and *per* se virus removal. As such, the term "virus inactivation" refers generally to the process of making a fluid disclosed herein completely free of infective viral contaminants. Any degree of viral inactivation using the methods disclosed herein is desirable. However, it is desirable to achieve the degree of viral inactivation necessary to meet strict safety guidelines for pharmaceuticals. These guidelines are set forth by the WHO and well known to those of skill in the art.

Detecting a viable lipid-coat containing virus can be accomplished by any technique that can qualitatively or quantitatively measure the presence or activity of a viable lipid-coat containing virus. Typically, a cell-culture based assay is used to determine titer levels of a virus, but *in vivo* infectivity assays can also be employed. Detection of virus amplification may be done, e.g., by microscopic examination (in case of a clearly visible cytopathogenic effect), a PCR-based detection assay, or an antibody-based detection assay. One non-limiting example is an *in vitro* infectivity assay called the Tissue Culture Infectious Dose 50 (TCID₅₀) assay. In this assay, fluid samples and serial dilutions thereof are dispensed into 96-well plates seeded with cells that can serve as hosts for the lipid-coat containing virus being assayed. After inoculation, the plates are incubated at a time and temperature sufficient to allow the virus to replicate in the host cells. After incubation, the cells are examined by microscope for signs of infection, such as, e.g., lysed cells, cells exhibiting a cytopathogenic effect, or any other criteria indicative of viral infection. From the pattern of positive (viral infection) and negative (no viral infection) wells the virus titer is calculated. The absence of any wells showing positive signs of infection is indicative of a fluid that is essentially free of a lipid-coat containing virus.

Another cell-culture based assay is a plaque assay, where virus-induced effects in the cell culture layer are visible or made visible macroscopically as plaques. The absence of any plaques is indicative of a fluid that is essentially free of a lipid-coat containing virus.

The present specification provides, in part, a mixture after incubation having a protein activity of at least 25% of the protein activity provided before incubation with the organic solvent and surfactant as disclosed herein. In aspects thereof, a mixture after incubation that has a protein activity of, e.g., about 25%, about 30%, about 40%, about 50%, about 60%, about 70%, about 80%, about 90%, or about 100%,of the protein activity provided before incubation. In other aspects thereof, a mixture after incubation that has a protein activity of, e.g., at least 30%, at least 40%, at least 50%, at least 60%, at least 70%, at least 75%, at least 80%, at least 85%, at least 90%, or at least 95% of the protein activity provided before incubation. In yet other aspects thereof, a mixture after incubation that has a protein activity of between, e.g., about 25% to about 100%, about 30% to about 100%, about 40% to about 100%, about 50% to about 100%, about 60% to about 100%, about 70% to about 100%, about 75% to about 100%, about 80% to about 100%, about 85% to about 100%, about 90% to about 100%, about 25% to about 95%, about 30% to about 95%, about 40% to about 95%, about 50% to about 95%, about 60% to about 95%, about 70% to about 95%, about 75% to about 95%, about 80% to about 95%, about 85% to about 95%, about 90% to about 95%, about 25% to about 90%, about 30% to about 90%, about 40% to about 90%, about 50% to about 90%, about 60% to about 90%, about 70% to about 90%, about 75% to about 90%, about 80% to about 90%, or about 85% to about 90% of the protein activity provided before incubation.

The present specification provides, in part, a protein activity present in the mixture after incubation is, e.g., at least 25% of the protein's activity provided before incubation with the organic solvent and surfactant as disclosed herein. In aspects thereof, a protein activity present in the mixture after incubation is, e.g., about 25%, about 30%, about 40%, about 50%, about 60%, about 70%, about 80%, about 90%, or about 100%,of the protein activity provided before incubation. In other aspects thereof, a protein activity present in the mixture after incubation is, e.g., at least 30%, at least 40%, at least 50%, at least 60%, at least 70%, at least 75%, at least 80%, at least 85%, at least 90%, or at least 95% of the protein activity provided before incubation. In yet other aspects thereof, a protein activity present in the mixture after incubation is between, e.g., about 25% to about 100%, about 30% to about 100%, about 40% to about 100%, about 50% to about 100%, about 60% to about 100%, about 70% to about 100%, about 75% to about 100%, about 80% to about 100%, about 85% to about 100%, about 90% to about 100%, about 25% to about 95%, about 30% to about 95%, about 40% to about 95%, about 50% to about 95%, about 60% to about 95%, about 70% to about 95%, about 75% to about 95%, about 80% to about 95%, about 85% to about 95%, about 90% to about 95%, about 25% to about 90%, about 30% to about 90%, about 40% to about 90%, about 50% to about 90%, about 60% to about 90%, about 70% to about 90%, about 75% to about 90%, about 80% to about 90%, or about 85% to about 90% of the protein activity provided before incubation.
In the present invention as defined by the appended claims, the Factor VIII after incubation has an activity of at least 25% of the activity provided.

Detecting an activity of a protein can be accomplished by any assay that can qualitatively or quantitatively measure a characteristic indicative of an activity associated with the protein being monitored, including, without limitation, a non-specific protein assay, such as, e.g., UV absorption or a chemical-based assay like a Bradford assay; or a specific protein assay, such as, e.g., an *in vitro* assay, a cell-based assay, or an *in vivo* assay. The actual assay used to detect an activity of a protein as disclosed herein can be determined by a person of ordinary skill in the art by taking into account factors, including, without limitation, the protein being assayed, the amount of protein present in the mixture after incubation, the characteristic being assayed, and the preference of the person of ordinary skill in the art.

For example, a chromogenic assay based on the blood coagulation cascade can be used to detect Factor VIII activity. In this assay, thrombin-activated Factor VIII forms a complex with Factor IXa, and this complex subsequently activates Factor X. Activated Factor X activity can be accessed by the hydrolysis of a chromogenic substrate which liberates a chromogenic group like p-nitro-aniline (pNA). The initial rate of pNA release, as determined by a change in absorbance per minute measured at 405 nm in dOD, is proportional to the Factor Xa activity and subsequently to the Factor VIII activity in the sample. By using excess of Factor IXa, and Factor X, the rate of activation of Factor X is solely proportional to the amount of thrombin cleaved Factor VIII present in the sample. Alternatively, Factor IXa activity can be determined by altering conditions so that Factor VIII and Factor X are in excess, and as such, Factor IXa is rate limiting. Similarly, Factor X activity can be determined by altering conditions so that Factor VIII and Factor IXa are in excess, and as such, Factor X is rate limiting. Thus, Factor VIII activity, as well as Factor IXa and Factor X, can be detected using a chromogenic assay based on the blood coagulation cascade.

As another example, a one-stage clotting assay that applies the Partial Activated Partial Thromboplastin Time (APTT) can be used to detect Factor VIII activity. In this assay, samples comprising Factor VIII, along with CaCl₂, are added to Factor VIII deficient plasma in order to promote coagulation and the effect of this sample on APTT clotting time of the plasma is a measure of the Factor VIII activity. Activities of unknown samples are calculated by comparing the Factor VIII activity observed with a standard curve generated from known Factor VIII activity samples. This blood clotting assay may also be used for any other protein involved in the blood coagulation cascade by using a plasma deficient in the protein being assayed.

The present specification provides, in part, a mixture after incubation is essentially free of protein aggregates of the protein having activity i.e., of a Factor VIII having an activity as defined by the appended claims. As used herein, the term "essentially free of protein aggregates" means that only trace amounts of protein aggregates of the protein having activity (protein of interest) can be detected or confirmed by the instrument or process being used to detect or confirm the presence or activity of protein aggregates and that such trace amount of protein aggregates is insufficient to be deleterious to the health of the human being, such as, e.g., to invoke an immune response in an individual. In an aspect of this embodiment, a mixture after incubation is entirely free of protein aggregates of the protein having activity. As used herein, the term "entirely free of protein aggregates" means that the presence of protein aggregates of the protein having activity (protein of interest) cannot be detected or confirmed within the detection range of the instrument or process being used to detect or confirm the presence or activity of the protein aggregates. A fluid that is essentially free or entirely free of protein aggregates of the protein having activity (protein of interest) can be used to make a pharmaceutical composition that is safe to administer to a human being because the protein aggregates are insufficient to be deleterious to the health of the human being.

In aspects of this embodiment, a mixture after incubation has less than, e.g., about 10%, about 9%, about 8%, about 7%, about 6%, about 5%, about 4%, about 3%, about 2%, about 1%, about 0.9%, about 0.8%, about 0.7%, about 0.6%, about 0.5%, about 0.4%, about 0.3%, about 0.2%, about 0.1%, about 0.09%, about 0.08%, about 0.07%, about 0.06%, about 0.05%, about 0.04%, about 0.03%, about 0.02%, or about 0.01%, of the protein having an activity in an aggregate form. In other aspects of this embodiment, a mixture after incubation has less than between, e.g., about 1% to about 0.01%, 0.9% to about 0.01%, 0.8% to about 0.01%, 0.7% to about 0.01%, 0.6% to about 0.01%, 0.5% to about 0.01%, 0.4% to about 0.01%, 0.3% to about 0.01%, 0.2% to about 0.01%, or 0.1% to about 0.01%, of the protein having an activity in an aggregate form.

Detecting formation of protein aggregates can be accomplished by any assay that can qualitatively or quantitatively measure the presence or activity of protein aggregates associated with the protein of interest being monitored, including, without limitation, size exclusion chromatography in conjunction with a non-specific and/or specific protein assay disclosed herein. The actual assay used to detect protein aggregates as disclosed herein can be determined by a person of ordinary skill in the art by taking into account factors, including, without limitation, the protein being assayed, the amount of protein present in the mixture after incubation, the characteristic being assayed, and the preference of the person of ordinary skill in the art.

The methods disclosed herein (e.g., the method of the invention as defined by the appended claims) may further comprise a step of removing an organic solvent, a surfactant, and/or other reagent or component used in the methods disclosed herein from the mixture after incubation. After completion of the method disclosed herein, the organic solvent, surfactant, and/or other reagent or component may be removed. Commonly employed methods for removal of detergent include extraction, filtration, diafiltration, buffer exchange, affinity or ion-exchange chromatography, precipitation, or lyophilization. See, e.g., Current Protocols in Protein Science, "Conventional Chromatographic Separations," Ch. 8-9, (John Wiley & Sons Inc., Hoboken, N.J.), After removal, the amount of an organic solvent, a surfactant, and/or other reagent or component remaining is an amount that has substantially no long term or permanent detrimental effect when administered to a human being.

In an embodiment of the present invention as defined by the appended claims, the mixture after removal of an organic solvent, surfactant, and/or other reagent or component is essentially free of the organic solvent, surfactant, and/or other reagent or component. As used herein, the term "essentially free of an organic solvent, surfactant, and/or other reagent or component" means that only trace amounts of an organic solvent, surfactant, and/or other reagent or component can be detected or confirmed by the instrument or process being used to detect or confirm the presence or activity of the organic solvent, surfactant, and/or other reagent or component and that such trace amount of the organic solvent, surfactant, and/or other reagent or component has no long term or permanent detrimental effect when administered to a human being. In an aspect of this embodiment, a mixture after removal of an organic solvent, surfactant, and/or other reagent or component is entirely free of the organic solvent, surfactant, and/or other reagent or component. As used herein, the term "entirely free of an organic solvent, surfactant, and/or other reagent or component" means that the presence of an organic solvent, surfactant, and/or other reagent or component cannot be detected or confirmed within the detection range of the instrument or process being used to detect or confirm the presence or activity of the organic solvent, surfactant, and/or other reagent or component. A protein contained within a mixture that is essentially free or entirely free of an organic solvent, surfactant, and/or other reagent or component can be used to make a pharmaceutical composition that is safe to administer to a human being because the amount of organic solvent, surfactant, and/or other reagent or component is insufficient to be deleterious to the health of the human being.

In aspects of this embodiment, the amount of organic solvent remaining in a mixture is, e.g., about 1 ppm, about 3 ppm, about 5 ppm, about 10 ppm, about 15 ppm, about 20 ppm, about 25 ppm, about 30 ppm, or about 35 ppm. In other aspects of this embodiment, the amount of organic solvent remaining in a mixture is, e.g., no more than 1 ppm, no more than 3 ppm, no more than 5 ppm, no more than 10 ppm, no more than 15 ppm, no more than 20 ppm, no more than 25 ppm, no more than 30 ppm, or no more than 35 ppm. In yet other aspects of this embodiment, the amount of organic solvent remaining in a mixture is between, e.g., about 1 ppm to about 20 ppm, about 1 ppm to about 25 ppm, about 1 ppm to about 30 ppm, about 1 ppm to about 35 ppm, about 3 ppm to about 20 ppm, about 3 ppm to about 25 ppm, about 3 ppm to about 30 ppm, or about 3 ppm to about 35 ppm.

In aspects of this embodiment, the amount of surfactant remaining in a mixture is, e.g., about 1 ppm, about 3 ppm, about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, about 75 ppm, about 80 ppm, about 85 ppm, about 90 ppm, or about 100 ppm. In other aspects of this embodiment, the amount of surfactant remaining in a mixture is, e.g., no more than 1 ppm, no more than 10 ppm, no more than 20 ppm, no more than 30 ppm, no more than 40 ppm, no more than 50 ppm, no more than 60 ppm, no more than 70 ppm, no more than 80 ppm, no more than 90 ppm, or no more than 100 ppm. In yet other aspects of this embodiment, the amount of surfactant remaining in a mixture is between, e.g., about 1 ppm to about 25 ppm, about 1 ppm to about 50 ppm, about 1 ppm to about 75 ppm, about 1 ppm to about 100 ppm, about 5 ppm to about 25 ppm, about 5 ppm to about 50 ppm, about 5 ppm to about 75 ppm, about 5 ppm to about 100 ppm, about 10 ppm to about 25 ppm, about 10 ppm to about 50 ppm, about 10 ppm to about 75 ppm, or about 10 ppm to about 100 ppm.

In an aspect of this embodiment, the amount of organic solvent remaining in a mixture is no more than 35 ppm and the amount of surfactant remaining in a mixture is no more than 100 ppm. In another aspect of this embodiment, the amount of organic solvent remaining in a mixture is no more than 3 ppm and the amount of surfactant remaining in a mixture is no more than 10 ppm. In yet another aspect of this embodiment, the amount of organic solvent remaining in a mixture is about 1 ppm to about 35 ppm and the amount of surfactant remaining in a mixture is about 1 ppm to about 100 ppm.

In another aspect of this embodiment, a method disclosed herein does not comprise a step of removing an organic solvent from the mixture after incubation. In yet another aspect of this embodiment, a method disclosed herein does not comprise a step of removing a surfactant from the mixture after incubation.

The methods disclosed herein (e.g., the method of the invention as defined by the appended claims), may further comprise a step of removing a virus from the mixture after incubation. As used herein, the term "removing a virus" or "virus removal" refers to a process that depletes a virus from a mixture disclosed herein, such that the virus particles are effectively extracted from the mixture. The virus can be viable virus or an inactivated virus. Removal is typically accomplished by size exclusion chromatography or positive adsorption chromatography where the protein of interest binds to a chromatographic resin. After removal, the amount of a virus remaining is an amount that has substantially no long term or permanent detrimental effect when administered to a human being.

In one embodiment, a mixture after removal of virus is essentially free of the virus. As used herein, the term "essentially free of a virus" means that only trace amounts of a virus can be detected or confirmed by the instrument or process being used to detect or confirm the presence or activity of the virus and that such trace amount of the virus is insufficient to be deleterious to the health of the human being. In an aspect of this embodiment, a mixture after removal of virus is entirely free of the virus. As used herein, the term "entirely free of a virus" means that the presence of virus cannot be detected or confirmed within the detection range of the instrument or process being used to detect or confirm the presence or activity of the virus. A protein contained within a mixture that is essentially free or entirely free of a virus can be used to make a pharmaceutical composition that is safe to administer to a human being because the virus is insufficient to be deleterious to the health of the human being.

In other aspects of this embodiment, a mixture after removal of virus comprises less than 1 x 10¹ PFU/mL of a virus, such as, e.g., less than 1 x 10⁰ PFU/mL of a virus, less than 1 x 10⁻¹ PFU/mL of a virus, 1 x 10⁻² PFU/mL of a virus, or 1 x 10⁻³ PFU/mL of a virus.

In yet other aspects of this embodiment, a mixture after removal of virus comprises less than an ID₅₀ for a virus, such as, e.g., at least 10-fold less than the ID₅₀ for a virus, at least 100-fold less than the ID₅₀ for a virus, at least 200-fold less than the ID₅₀ for a virus, at least 300-fold less than the ID₅₀ for a virus, at least 400-fold less than the ID₅₀ for a virus, at least 500-fold less than the ID₅₀ for a virus, at least 600-fold less than the ID₅₀ for a virus, at least 700-fold less than the ID₅₀ for a virus, at least 800-fold less than the ID₅₀ for a virus, at least 900-fold less than the ID₅₀ for a virus, or at least 1000-fold less than the ID₅₀ for a virus.

In another aspect of this embodiment, a method disclosed herein does not comprise a step of removing a virus from the mixture after incubation.

### EXAMPLES

The following examples are provided for illustrative purposes only in order to facilitate a more complete understanding

### Example 1

### Assessment of Viral Inactivation

This example illustrates that the methods for inactivating a lipid-coat containing virus disclosed herein result in a mixture after incubation is essentially free of a viable lipid-coat containing virus.

A recombinant Factor VIII, produced by a CHO cell line that secretes the protein into the cell culture medium, was harvested by collecting the medium and centrifuging it to remove cellular debris. In a cold room kept at or below 10°C, the harvested supernatant was diluted and filtered through a 0.2 µm filter, and the Factor VIII captured by passing through an immunoaffinity chromatography column comprising immobilized α-Factor VIII mouse monoclonal antibodies and collecting the elute. The Factor VIII was further processed by passing the immunoaffinity chromatography eluate through a cation exchange chromatography column comprising negatively charged sulfonated groups. The collected eluate from this exchange column was then cooled to either about 2°C or about 10°C.

The cooled eluate was then divided into three aliquots and the lipid-coated model virus Pseudorabies virus was added at a ratio of 1:13 (e.g., 24 mL process feed plus 2 mL of virus stock). The aliquots were then mixed with a solvent/detergent solution, and chilled to the same temperature, as follows: Aliquot 1, 0.21% (v/v) Tri(n-butyl)phosphate (TNBP), 0.7% (v/v) polyoxyethylene octyl phenyl ether (TRITON^{®} X-100) and 0.21% (v/v) polysorbate 80 sorbitan monooleate (TWEEN^{®} 80); Aliquot 2, 0.06% (v/v) Tri(n-butyl)phosphate (TNBP), 0.2% (v/v) polyoxyethylene octyl phenyl ether (TRITON^{®} X-100) and 0.06% (v/v) polysorbate 80 sorbitan monooleate (TWEEN^{®} 80); and Aliquot 3, 0.03% (v/v) Tri(n-butyl)phosphate (TNBP), 0.1% (v/v) polyoxyethylene octyl phenyl ether (TRITON^{®} X-100) and 0.03% (v/v) polysorbate 80 sorbitan monooleate (TWEEN^{®} 80). The component concentrations of the solvent/detergent solutions equate to 70%, 20% and 10% of the nominal concentration of these components during routine manufacturing, respectively. These sub-optimal concentrations were used to evaluate robustness of the method by using conditions least favorable for virus inactivation, as well as evaluate inactivation kinetics.

To access the effectiveness of the S/D treatment, the log₁₀ reduction of lipid-coated virus was determined. Samples from the virus-spiked starting material and during the S/D treatment were drawn; the virus inactivating effect of the solvent/detergent components was stopped in the samples by immediate dilution with cold cell culture medium before virus titration. Log reduction factors were calculated by subtracting the (log) titer of the final sample from the (log) titer of the spiked starting material; where no infectivity in the final sample could be observed, the limit of detection was taken for this calculation.

The results indicate that Pseudorabies virus inactivation occurred within about 1 to about 2 minutes at either about 2°C or about 10°C (Table 1). As the experimental runs were done at or below the lower limits of solvent/detergent component concentration and below the lower limits of S/D treatment duration, the reduction factors obtained are robust estimates of the virus inactivation capacity of a large-scale process. These results demonstrate that S/D treatments at about 10°C and at about 2°C were robust and very effectively and rapidly inactivated lipid-enveloped viruses.

| **Table 1. PRV virus inactivation** | | | | | | | | |
|---|---|---|---|---|---|---|---|---|
| **Solvent/detergent solution** | **Incubation Temperature** | | | | | | | |
| | **2°C** | | | | **10°C** | | | |
| | **Inactivation time (min)¹** | **Virus Reduction Factor (log₁₀)** | | | **Inactivation time (min)** | **Virus Reduction Factor (log₁₀)** | | |
| | | **Run 1** | **Run 2** | **Mean** | | **Run 1** | **Run 2** | **Mean** |
| 70% | 1-2 | >4.5 | >4.6 | >4.6 | 1-2 | >4.8 | >4.5 | >4.6 |
| 20% | 1-2 | >5.3 | ND² | ND | 1-2 | >5.0 | ND | ND |
| 10% | 1-2 | >5.7 | ND | ND | 1-2 | >5.5 | ND | ND |
| ¹ Time required for viral inactivation to below the limit of detection. | | | | | | | | |
| ² ND, Not determined. | | | | | | | | |

### Example 2

### Assessment of Viral Inactivation

This example illustrates that the methods for inactivating a lipid-coat containing virus disclosed herein result in a mixture after incubation is essentially free of a viable lipid-coat containing virus.

A recombinant Factor VIII was expressed and purified as essentially described in Example 1. The collected eluate from the cation exchange column was then cooled to about 2°C.

The cooled eluate was then divided into six aliquots and lipid-enveloped viruses were added to the aliquots at a ratio of 1:13 (e.g., 24 mL process feed plus 2 mL of virus stock) as follows: Aliquots 1 and 2, Pseudorabies virus (PRV) was added; Aliquots 3 and 4, Moloney murine leukemia virus (X-MuLV) was added; and Aliquots 5 and 6, bovine viral diarrhea virus (BVDV) was added. The aliquots were then mixed with a solvent/detergent solution, and chilled to about 2°C, as follows: Aliquots 1, 3 and 5; 0.21% (v/v) Tri(n-butyl)phosphate (TNBP), 0.7% (v/v) polyoxyethylene octyl phenyl ether (TRITON^{®} X-100) and 0.21% (v/v) polysorbate 80 sorbitan monooleate (TWEEN^{®} 80); and Aliquots 2, 4, and 6; 0.03% (v/v) Tri(n-butyl)phosphate (TNBP), 0.1% (v/v) polyoxyethylene octyl phenyl ether (TRITON^{®} X-100) and 0.03% (v/v) polysorbate 80 sorbitan monooleate (TWEEN^{®} 80). The component concentrations of the solvent/detergent solutions equate to 70% and 10% of the nominal concentration of these components at routine manufacturing. These sub-optimal concentrations were used to evaluate robustness of the method by using conditions least favorable for virus inactivation, as well as evaluate inactivation kinetics.

To access the effectiveness of the S/D treatment, the log₁₀ reduction of each of the three lipid-coated viruses was determined. Samples from the virus-spiked starting material and during the S/D treatment were drawn; the virus inactivating effect of the solvent/detergent components was stopped in the samples by immediate dilution with cold cell culture medium before virus titration. Log reduction factors were calculated by subtracting the (log) titer of the final sample from the (log) titer of the spiked starting material; where no infectivity in the final sample could be observed, the limit of detection was taken for this calculation.

The results indicate that PRV, X-MuLV, and BVDV inactivation all occurred within about 1-2 minutes at about 2°C when incubated with a 70% solvent/detergent solution (Table 2). As the experimental runs were done at or below the lower limits of solvent/detergent component concentration and below the lower limits of S/D treatment duration, the reduction factors obtained are robust estimates of the virus inactivation capacity of a large-scale process. These results demonstrate that S/D treatments at about 2°C were robust and very effectively and rapidly inactivated lipid-enveloped viruses.

| **Table 2. PRV, X-MuLV, and BVDV inactivation** | | | |
|---|---|---|---|
| **Virus** | **Solvent/detergent solution** | **Inactivation time (min)¹** | **Virus Reduction Factor (log₁₀)** |
| PRV | 10% | 10 | >5.2 |
| | 70% | 1-2 | >4.1 |
| | 70% | 1-2 | >4.5 |
| X-MuLV | 10% | >60 | 2.7 |
| | 70% | 1-2 | >4.9 |
| | 70% | 1-2 | >3.8 |
| BVDV | 10% | >60 | 2.4 |
| | 70% | 1-2 | >5.3 |
| | 70% | 1-2 | >5.1 |
| ¹ Time required for viral inactivation to below the limit of detection. | | | |

### Example 3 (Reference Example)

### Assessment of Mixing Time, Filtration, and Protein Yield

This example illustrates that the methods for inactivating a lipid-coat containing virus disclosed herein have no negative impact on the mixing time of the solvent/detergent components, retention of solvent/detergent components during filtration, and protein filtration yield.

In order to evaluate the influence of a lower temperature on the mixing time of solvent/detergent components, 50 mL solvent/detergent solutions were prepared by adding 0.3% (v/v) TNBP, 1.0% (v/v) polyoxyethylene octyl phenyl ether (TRITON^{®} X-100) and 0.3% (v/v) polysorbate 80 sorbitan monooleate (TWEEN^{®} 80) and stirring at 4 ± 2°C for 10, 20, or 30 minutes. As the solvent/detergent solution already contained 0.1% polysorbate 80 sorbitan monooleate (TWEEN^{®} 80), the final concentration for this compound was of 0.4%. The concentration of TNBP and polyoxyethylene octyl phenyl ether (TRITON^{®} X-100) were determined simultaneously by using C18 RP-HPLC with a refractive index detector (RID). The concentration of polysorbate 80 sorbitan monooleate (TWEEN^{®} 80) was determined by C1 RP-HPLC using an evaporative light scattering detector (ELSD). For the HPLC analysis of TNBP and polyoxyethylene octyl phenyl ether (TRITON^{®} X-100), an isocratic elution is employed using 68% methanol in water. In the assay of polysorbate 80 sorbitan monooleate (TWEEN^{®} 80), a gradient elution from 60% methanol in water to 100% methanol is used.

| **Table 3. Mixing time effects on solvent/detergent components** | | | |
|---|---|---|---|
| **Time (min)** | **S/D component concentration (%)** | | |
| | **TNBP** | **TRITON X-100** | **Tween 80** |
| 10 | 0.36 | 1.2 | 0.42 |
| 20 | 0.35 | 1.1 | 0.40 |
| 30 | 0.37 | 1.2 | 0.44 |
| Mean | 0.36 ± 0.01 | 1.2 ± 0.06 | 0.42 ± 0.02 |

These results confirm that the concentration of TNBP, polyoxyethylene octyl phenyl ether (TRITON^{®} X-100) and polysorbate 80 sorbitan monooleate (TWEEN^{®} 80) stabilized rapidly and within 10 minutes and that a low temperature of 4°C ± 2°C had no negative impact on the mixing time of the solvent/detergent components (Table 3).

To evaluate the impact of the temperature toward a potential retention of solvent/detergent conponents during the filtration step, 50 mL solvent/detergent solutions were prepared by adding 0.3% (v/v) TNBP, 1.0% (v/v) polyoxyethylene octyl phenyl ether (TRITON^{®} X-100) and 0.3% (v/v) polysorbate 80 sorbitan monooleate (TWEEN^{®} 80), stirring at 4 ± 2°C for 30 minutes and the concentration of each component was measured as described above. The solvent/detergent solutions were then processed under three different temperature conditions: 22.5°C with a warm up period of about 8 hours; 22.5°C with a warm up period of about 2 hours; and 4°C without any warm-up or preheating step. At the end of the incubation period, samples were filtrated through a 0.2 µm filter and TNBP, polyoxyethylene octyl phenyl ether (TRITON^{®} X-100) and polysorbate 80 sorbitan monooleate (TWEEN^{®} 80) concentrations were measured by HPLC.

| **Table 4. Temperature effects on solvent/detergent components** | | | | | | | |
|---|---|---|---|---|---|---|---|
| **Temperature Condition (n=3)** | **Run** | **S/D component concentration (%) before 0.2 µm filtration** | | | **S/D component concentration (%) after 0.2 µm filtration** | | |
| | | **TNBP** | **TRITON^{®} X-100** | **TWEEN^{®} 80** | **TNBP** | **TRITON^{®} X-100** | **TWEEN^{®} 80** |
| 22.5°C / 2H | 1 | 0.28 | 1.00 | 0.41 | 0.28 | 1.00 | 0.38 |
| | 2 | 0.28 | 1.00 | 0.42 | 0.29 | 1.10 | 0.44 |
| | 3 | 0.27 | 1.00 | 0.41 | 0.28 | 1.00 | 0.41 |
| | Mean | 0.28 | 1.00 | 0.41 | 0.28 | 1.03 | 0.41 |
| | | | | | | | |
| 22.5°C / 8H | 1 | 0.23 | 0.80 | 0.33 | 0.28 | 1.00 | 0.40 |
| | 2 | 0.28 | 1.00 | 0.39 | 0.28 | 1.00 | 0.39 |
| | 3 | 0.28 | 1.00 | 0.42 | 0.25 | 0.90 | 0.37 |
| | Mean | 0.26 | 0.93 | 0.38 | 0.27 | 0.97 | 0.39 |
| | | | | | | | |
| 4°C / 2H | 1 | 0.27 | 1.00 | 0.38 | 0.27 | 1.00 | 0.40 |
| | 2 | 0.28 | 1.00 | 0.40 | 0.28 | 1.00 | 0.41 |
| | 3 | 0.27 | 1.00 | 0.42 | 0.27 | 1.00 | 0.41 |
| | Mean | 0.27 | 1.00 | 0.40 | 0.27 | 1.00 | 0.41 |

The results indicate that none of the solvent/detergent components were retained onto the 0.2 µm filter (Table 4). In addition, the lower incubation temperature (4°C ± 2°C) had no negative impact on the filterability of the solvent/detergent components.

To evaluate the influence of a lower temperature on protein activity recovered, 50 mL solvent/detergent solutions were prepared by adding 0.3% (v/v) TNBP, 1.0% (v/v) polyoxyethylene octyl phenyl ether (TRITON^{®} X-100) and 0.3% (v/v) polysorbate 80 sorbitan monooleate (TWEEN^{®} 80), stirring at 4 ± 2°C for 30 minutes. The solvent/detergent solutions were then processed under three different temperature conditions: 22.5°C with a warm up period of about 8 hours; 22.5°C with a warm up period of about 2 hours; and 4°C without any warm-up or preheating step. At the end of the incubation period, samples were filtrated through a 0.2 µm filter and Factor VIII activity was measured using a chromogenic substrate assay before and after filtration.

To perform a chromogenic assay of Factor VIII activity, an aliquot of solvent/detergent treated solution is pre-diluted in sample dilution buffer (50 mM Tris, 5 mM CaCl₂, 225 mM NaCl, 0.1% polysorbate 80 sorbitan monooleate (TWEEN^{®} 80), pH 6.7 ± 0.2) to approximately 25 IU/mL and then further diluted to 1 IU/mL in Factor VIII depleted plasma. A 100µL prediluted sample is then mixed with 0.03 M CaCl₂, 0.06 mM phospholipids, 100µL of 0.3 µM Factor IXa, 100µL of 1µM Factor X, and 500µL of 3.4 µM of chromogenic substrate CH₃OCO-D-cyclohexylglycyl-glycyl-arginyl-p-nitroanilide to ensure that the Factor VIII is the rate limiting component of the reaction. The mixture was incubated at 37°C for 90 seconds and then spectrophotometer readings made at 405 nm were taken to determine the rate of chromogenic substrate hydrolysis and release of p-nitro-aniline.

The Factor VIII activity measured were equivalent before and after the 0.2 µm filtration step. This result confirms that the lower temperature has no negative impact on Factor VIII recovery after 0.2 µm filtration step, in absence of the S/D components.

| **Table 5. Temperature effects on Factor VIII Activity** | | |
|---|---|---|
| **Temperature condition (n=3)** | **Factor VIII Activity before 0.2 µm filtration (Ul/mL)** | **Factor VIII Activity after 0.2 µm filtration (Ul/mL)** |
| 22.5°C / 2H | 2960 ± 199 | 3003 ± 369 |
| 22.5°C / 8H | 2965 ± 303 | 3069 ± 201 |
| 4°C / 2H | 2806 ± 314 | 2787 ± 198 |

The results indicate that Factor VIII recovery was about 100% for all temperature conditions evaluated and that protein activities were equivalent before and after the filtration step despite the presence of the solvent/detergent components (Table 5).

### Example 4 (Reference Example)

### Inactivating a Lipid-Coat Containing Virus in Fluid Comprising a Protein Having Activity

This example illustrates the methods disclosed herein for inactivating a lipid-coat containing virus in a fluid comprising a protein with an activity.

To evaluate the overall effectiveness of the method for inactivating a lipid-coat containing virus disclosed herein on protein activity recovered, 50 mL solvent/detergent solutions were prepared by adding 0.3% (v/v) TNBP, 1.0% (v/v) polyoxyethylene octyl phenyl ether (TRITON^{®} X-100) and 0.3% (v/v) polysorbate 80 sorbitan monooleate (TWEEN^{®} 80), stirring at 4 ± 2°C for 30 minutes. The solvent/detergent solutions were then processed under three different temperature conditions: 22.5°C with a warm up period of about 8 hours; 22.5°C with a warm up period of about 2 hours; and 4°C without any warm-up or preheating step. At the end of the incubation period, samples were purified using ion exchange column chromatography and Factor VIII activity was measured using a chromogenic substrate assay before and after filtration essentially as described in Example 3.

The results indicate that mean recovery yields for Factor VIII were 83.02% with the solvent/detergent treatment carried out at 4°C for two hours; 82.83% for treatment carried out at 22.5°C for two hours; and 80.93% for treatment carried out at 22.5°C for eight hours (Table 6).

These results confirm that the solvent/detergent treatment carried out at 4°C for two hours does not significantly impact the recovery of active Factor VIII. Moreover, the specific activity of Factor VIII appears better preserved when the incubation was carried out at 4°C instead of 22.5°C. About 6% of specific activity were lost when the solvent/detergent treatment were done at 22.5°C, whereas only a slight decrease in the specific activity of about 1% was observed in Factor VIII when treatment was done at 4°C. Therefore, an unexpected increase in the Factor VIII mean recovery was observed with the solvent/detergent treatment carried out at a low temperature.

| **Table 6. Factor VIII Activity And Yields Using Method For Inactivating a Lipid-Coat Containing Virus.** | | | | | | | | | | | | | | | | |
|---|---|---|---|---|---|---|---|---|---|---|---|---|---|---|---|---|
| **Temperature Condition (n=3)** | **Run** | **Fluid before solvent/detergent treatment** | | | | | | | **Elution pool after solvent/detergent treatment** | | | | | | | **Recovery (%)** |
| | | **TSP (µg/mL)** | **FVIII (UI/mL)** | **SpAct (UI/mg)** | **Vol (mL)** | **TSP (µg/mL)** | **FVIII (UI)** | **SpAct (UI/mg)** | **TSP (µg/mL)** | **FVIII (UI/mL)** | **SpAct (Ul/mg)** | **Vol (mL)** | **TSP (µg/mL)** | **FVIII (UI)** | **SpAct (Ul/mg)** | |
| 22.5°C / 2H | 1 | 484 | 2959 | 6121 | 24.92 | 12061 | 73738 | 6114 | 1226 | 8475 | 6750 | 8.26 | 10375 | 70004 | 6748 | 94.94 |
| | 2 | 473 | 3307 | 6991 | 24.90 | 11778 | 82344 | 6992 | 1337 | 8308 | 6214 | 7.67 | 10255 | 63772 | 6214 | 77.39 |
| | 3 | 469 | 3077 | 6561 | 24.91 | 11683 | 766648 | 6561 | 1362 | 7613 | 5591 | 7.67 | 10447 | 58392 | 5590 | 76.18 |
| | Mean | | | | | 11841 | 77577 | 6555 | | | | | 10359 | 64039 | 6184 | 82.83 |
| | | | | | | | | | | | | | | | | |
| 22.5°C / 8H | 1 | 491 | 3096 | 6305 | 24.90 | 12226 | 77090 | 6305 | 1242 | 7975 | 6421 | 8.26 | 10259 | 65874 | 6421 | 85.45 |
| | 2 | 479 | 3162 | 6608 | 24.91 | 11932 | 78765 | 6601 | 1265 | 8772 | 6937 | 8.26 | 10449 | 72457 | 6934 | 91.99 |
| | 3 | 470 | 3007 | 6398 | 24.92 | 11712 | 74934 | 6398 | 1335 | 6385 | 4785 | 7.67 | 10239 | 48973 | 4783 | 65.35 |
| | Mean | | | | | 11957 | 76930 | 6435 | | | | | 10316 | 62434 | 6046 | 80.93 |
| | | | | | | | | | | | | | | | | |
| 4°C / 2H | 1 | 491 | 2904 | 5920 | 24.90 | 12226 | 72310 | 5914 | 1218 | 8085 | 6640 | 8.26 | 10061 | 66782 | 6638 | 92.36 |
| | 2 | 475 | 3082 | 6495 | 24.89 | 11823 | 76711 | 6488 | 1183 | 7808 | 6603 | 8.26 | 9772 | 64494 | 6600 | 84.07 |
| | 3 | 467 | 3027 | 6483 | 24.87 | 11614 | 75281 | 6482 | 1309 | 7129 | 5448 | 7.67 | 10040 | 54679 | 5446 | 72.63 |
| | Mean | | | | | 11888 | 74767 | 6295 | | | | | 9957 | 61985 | 6228 | 83.02 |
| TSP, Total soluble protein determined using a UV-VIS spectrophotometer and absorbance at 285 nm and 350 nm. | | | | | | | | | | | | | | | | |
| FVIII, Factor VIII | | | | | | | | | | | | | | | | |
| SpAct, Specific activity, Factor VIII activity per mg of total soluble protein. | | | | | | | | | | | | | | | | |
| Vol, volume. | | | | | | | | | | | | | | | | |

### Example 5

### Assessment of Protein Aggregation

This example illustrates that the methods for inactivating a lipid-coat containing virus disclosed herein prevented protein aggregation.

To assess the impact of a virus inactivation step at a low temperature on protein aggregate formation, solvent/detergent treatments were carried out under three different temperature conditions: 22.5°C with a warm up period of about 8 hours; 22.5°C with a warm up period of about 2 hours; and 4°C without any warm-up or preheating step. In all condition, solvent/detergent reagents were added when the target temperature was reached. After the incubation period, set to 1 hour for all temperature conditions, solvent/detergent components were removed by ion exchange chromatography and aggregate amounts measured in final bulks. All solvent/detergent conditions were repeated three times.

The determination of aggregates (expressed in percentage) was carried out by size exclusion chromatography (SEC) on an HPLC system equipped with a Bio-Sep-SEC-S4000 column. The stationary phase contains hydrophilic groups bonded to a silica support that is suitable for separating proteins in the molecular weight range from 15 to 2,000 kDa. Samples taken from solvent/detergent treated solutions were diluted down to 50 µg/mL and 11 µL were injected onto the column. Elution buffer was 20 mM Tris, 250mM NaCl, 3 mM CaCl₂ dihydrate, 0.05% (v/v) polysorbate 80 sorbitan monooleate (TWEEN^{®} 80), 7.5 % (v/v) ethylene glycol, pH adjusted to 7.0 ± 0.2. Separation was done under an isocratic mobile phase buffer. Detection was done using a fluorescence detector at an excitation wavelength of 285 nm and an emission wavelength of 335 nm. The response is proportional to the amount of proteins present in the different peaks of the obtained chromatogram. Percentages of the aggregates are computed as a ratio of fraction peak area to total peak area (Table 7).

| **Table 7. Aggregate formation** | |
|---|---|
| **Temperature condition (n=3)** | **Mean aggregate formation (Specification < 1%)** |
| 22.5°C / 2H | 0.7 ± 0.13 |
| 22.5°C / 8H | 0.6 ± 0.07 |
| 4°C / 2H | Not detectable |

Unexpectedly, the experiments carried out at 4°C lead to an undetectable (< 0.25%) aggregate amount in the final bulk eluate, whereas under the processes carried out at 22.5°C, much larger aggregate formation was detected (0.7% and 0.6%). The reduction of aggregates in a final bulk is significant as aggregates are usually recognized to enhance immune responses against monomeric forms of a protein.

### Example 6 (Reference Example)

### Inactivating a Lipid-Coat Containing Virus in Fluid Comprising a Protein Having Activity

This example illustrates the methods disclosed herein for inactivating a lipid-coat containing virus in a fluid comprising a protein with an activity.

A recombinant Factor VIII, produced by a CHO cell line that secretes the protein into the cell culture medium is grown in 2,500 liter bioreactors and is harvested by chilling to 10°C, collecting the medium, and centrifuging it to remove cellular debris. In a cold room kept at or below 10°C, the harvested supernatant is diluted and filtered through a 0.2 µm filter, and the Factor VIII is captured by passing through an immunoaffinity chromatography column comprising immobilized α-Factor VIII mouse monoclonal antibodies and collecting the elute. The Factor VIII is further processed by passing the immunoaffinity chromatography eluate through a cation exchange chromatography column comprising negatively charged sulfonated groups. About 8 liters of collected eluate from this exchange column is then cooled to about 4°C and mixed for about 10 minutes with a solvent/detergent solution, chilled to the same temperature, in an amount of 16.6 g of solvent/detergent solution per kg of cation exchanger elute. The solvent/detergent solution comprises tri(n-butyl) phosphate (TNBP), polyoxyethylene octyl phenyl ether (TRITON^{®} X-100), and polysorbate 80 sorbitan monooleate (TWEEN^{®} 80) in a ratio of 18.3:66.3:20.2 by weight. This equates to a final concentration of about 0.3% (v/v) Tri(n-butyl)phosphate (TNBP), about 1.0% (v/v) polyoxyethylene octyl phenyl ether (TRITON^{®} X-100) and about 0.3% (v/v) polysorbate 80 sorbitan monooleate (TWEEN^{®} 80). After continuous mixing at about 4°C for no more than about 120 minutes, the solvent/detergent treated elute is then filtered through a 0.2 µm filter to remove viral debris. The filtered solvent/detergent treated solution is diluted with anion exchange chromatography equilibration buffer and further processed by passing through an anion exchange chromatography column to remove the solvent/detergent components. The final purified solution comprising Factor VIII is collected from the anion exchange chromatography elution fractions. The presence of lipid-coat containing viruses, Factor VIII activity, amount of aggregates, and trace amount of solvent/detergent components are then measured in the final purified solution comprising Factor VIII.

To assay for the presence or activity of a viable lipid-coat containing virus in the final purified solution comprising Factor VIII, a TCID₅₀ assay was performed. A sample of filtered solvent/detergent treated solution was drawn and diluted 1:00 or 1:10 with Vero cell culture medium. Serial 0.5 log₁₀ dilutions of samples were prepared in Vero cell culture medium and 100 µL of each dilution were added to each of 8 wells of a microtiter plate column seeded with Vero cells. The microtiter plates were incubated in a humidified and CO₂-regulated incubator at 36 ± 2°C for 7 days. After incubation, the cells were examined by microscope for signs of infection, such as, e.g., lysed cells, cells exhibiting a cytopathogenic effect, or any other criteria indicative of viral infection. From the pattern of positive (viral infection) and negative (no viral infection) wells the virus titer was calculated according to the *Poisson* distribution and expressed as PFU/mL and log₁₀ [TCID₅₀/mL]. The absence of positive signs of infection in any wells indicates that the treated solution is essentially free of a lipid-coat containing virus.

To assay for the amount of Factor VIII activity present in the final purified solution, a chromogenic assay of Factor VIII activity is performed. An aliquot of filtered solvent/detergent treated solution is pre-diluted in sample dilution buffer (50 mM Tris, 5 mM CaCl₂, 225 mM NaCl, 0.1% polysorbate 80 sorbitan monooleate (TWEEN^{®} 80), pH 6.7 ± 0.2) to approximately 25 IU/mL and then further diluted to 1 IU/mL in Factor VIII depleted plasma. A 100µL prediluted sample is then mixed with 0.03 M CaCl₂, 0.06 mM phospholipids, 100µL of 0.3 µM Factor IXa, 100µL of 1µM Factor X, and 500µL of 3.4 µM of chromogenic substrate CH₃OCO-D-cyclohexylglycyl-glycyl-arginyl-*p*-nitroanilide to ensure that the Factor VIII is the rate limiting component of the reaction. The mixture is incubated at 37°C for 90 seconds and then spectrophotometer readings made at 405 nm are taken to determine the rate of chromogenic substrate hydrolysis and release of p-nitro-aniline.

To assay for the amount of Factor VIII activity present in the final purified solution, a one-stage coagulation assay of Factor VIII activity was performed. An aliquot of filtered solvent/detergent treated solution was pre-diluted in sample dilution buffer (50 mM Tris, 5 mM CaCl₂, 225 mM NaCl, 0.1% polysorbate 80 sorbitan monooleate (TWEEN^{®} 80), pH 6.7 ± 0.2) to approximately 25 IU/mL and then further diluted to 1 IU/mL in Factor VIII depleted plasma. A 100µL of prediluted sample was then mixed with 100µL of Factor VIII deficient plasma. The mixture was incubated at 37°C for 3 minutes and 100µL of 25 mM CaCl₂ is added to start the coagulation process. The amount of Factor VIII present was determined by comparing the APTT clotting time value with a standard curve calculated by an instrument running a calculation program that makes a double logarithmic plot of the clotting time versus the Factor VIII concentration. The coagulation analyzer and proper reagents for coagulation assays are designed to measure the activity of coagulation parameters in physiologic range. The results indicate that the mean specific activity for Factor VIII was about 6,120 UI/mg. This mean specific activity is higher than 5,809 UI/mg, the mean specific activity for Factor VIII processed using a viral inactivation method that incubates the fluid in a organic solvent/surfactant mixture at abut 20°C to about 25°C.

To assay for the amount of protein aggregates present in the final purified solution comprising Factor VIII, size exclusion chromatography and UV absorption experiments are done essentially as described in Example 5.

To assay for trace amount of solvent/detergent components present in the final purified solution comprising Factor VIII, RP-HPLC experiments are done essentially as described in Example 3.

In closing, it is to be understood that although aspects of the present specification are highlighted by referring to specific embodiments, one skilled in the art will readily appreciate that these disclosed embodiments are only illustrative of the principles of the subject matter disclosed herein. As such, various modifications or changes to or alternative configurations of the disclosed subject matter can be made in accordance with the teachings herein. Lastly, the terminology used herein is for the purpose of describing particular embodiments only, and is not intended to limit the scope of the present invention, which is defined solely by the claims. Accordingly, the present invention is defined by the appended claims.

Certain embodiments of the present invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the present invention to be practiced otherwise than specifically described herein.

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses a range of plus or minus ten percent above and below the value of the stated characteristic, item, quantity, parameter, property, or term. Accordingly, unless indicated to the contrary, the numerical parameters set forth in the specification and attached claims are approximations that may vary. At the very least, and not as an attempt to limit the application of the doctrine of equivalents to the scope of the claims, each numerical indication should at least be construed in light of the number of reported significant digits and by applying ordinary rounding techniques. Notwithstanding that the numerical ranges and values setting forth the broad scope of the invention are approximations, the numerical ranges and values set forth in the specific examples are reported as precisely as possible. Any numerical range or value, however, inherently contains certain errors necessarily resulting from the standard deviation found in their respective testing measurements. Recitation of numerical ranges of values herein is merely intended to serve as a shorthand method of referring individually to each separate numerical value falling within the range.

The terms "a," "an," "the" and similar referents used in the context of describing the present invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the present invention and does not pose a limitation on the scope of the invention otherwise claimed. No language in the present specification should be construed as indicating any non-claimed element essential to the practice of the invention.

## Claims

1. A method of inactivating a pseudorabies virus, the method comprising the steps of: a) providing a fluid comprising a Factor VIII having an activity; b) mixing tri-(n-butyl)phosphate, polyoxyethylene octyl phenyl ether, and polysorbate 80 sorbitan monooleate with the fluid, thereby creating a mixture, wherein the final concentration of tri(n-butyl)phosphate is 0.21% (v/v), the final concentration of polyoxyethylene octyl phenyl ether is 0.7% (v/v), and the final concentration of polysorbate 80 sorbitan monooleate is 0.21% (v/v); and c) incubating the mixture for no more than 120 minutes; wherein both steps (b) and (c) are performed at a temperature of between 2°C to 8°C; wherein the mixture after incubation is essentially free of a viable pseudorabies virus; and wherein the Factor VIII after incubation has an activity of at least 25% of the activity provided in step (a).

2. The method of claim 1, wherein the Factor VIII after incubation has an activity of at least 50% of the activity provided in step (a).

3. The method of claim 1, wherein the Factor VIII after incubation has an activity of at least 75% of the activity provided in step (a).

## Patentansprüche

1. Verfahren zum Inaktivieren eines Pseudorabiesvirus, wobei das Verfahren die folgenden Schritte umfasst: a) Bereitstellen eines Fluids, umfassend einen Faktor VIII, der eine Aktivität aufweist; b) Mischen von Tri(n-butyl)phosphat, Polyoxyethylenoctylphenylether und Polysorbat-80-Sorbitanmonooleat mit dem Fluid, wodurch ein Gemisch geschaffen wird, wobei die Endkonzentration von Tri(n-butyl)phosphat 0,21 Vol.-%, die Endkonzentration von Polyoxyethylenoctylphenylether 0,7 Vol.-% und die Endkonzentration von Polysorbat-80-Sorbitanmonooleat 0,21 Vol.-% beträgt; und c) Inkubieren der Mischung für nicht mehr als 120 Minuten; wobei beide Schritte (b) und (c) bei einer Temperatur zwischen 2 °C und 8 °C durchgeführt werden; wobei die Mischung nach Inkubation im Wesentlichen frei von einem lebensfähigen Pseudorabiesvirus ist; und wobei der Faktor VIII nach Inkubation eine Aktivität von mindestens 25 % der in Schritt (a) bereitgestellten Aktivität aufweist.

2. Verfahren nach Anspruch 1, wobei der Faktor VIII nach Inkubation eine Aktivität von mindestens 50 % der in Schritt (a) bereitgestellten Aktivität aufweist.

3. Verfahren nach Anspruch 1, wobei der Faktor VIII nach Inkubation eine Aktivität von mindestens 75 % der in Schritt (a) bereitgestellten Aktivität aufweist.

## Revendications

1. Procédé d'inactivation d'un virus de la pseudorage, le procédé comprenant les étapes suivantes : a) la fourniture **d'un** fluide présentant un facteur VIII actif ; b) le mélange de phosphate de tri(n-butyle), d'éther de polyoxyéthylène octylphényle et de monooléate de polysorbate 80 sorbitane avec le fluide, créant ainsi un mélange, dans lequel la concentration finale en phosphate de tri(n-butyle) est de 0,21 % (v/v), la concentration finale en éther de polyoxyéthylène octylphényle est de 0,7 % (v/v) et la concentration finale en monooléate de polysorbate 80 sorbitane est de 0,21 % (v/v) ; et c) l'incubation du mélange pendant 120 minutes maximum ; dans lequel les étapes (b) et (c) sont réalisées à une température comprise entre 2 °C et 8 °C ; dans lequel le mélange après incubation est essentiellement exempt de virus de la pseudorage viable ; et dans lequel le facteur VIII après incubation présente une activité d'au moins 25 % de l'activité fournie à l'étape (a).

2. Procédé selon la revendication 1, dans lequel le facteur VIII après incubation présente une activité d'au moins 50 % de l'activité fournie à l'étape (a).

3. Procédé selon la revendication 1, dans lequel le facteur VIII après incubation présente une activité d'au moins 75 % de l'activité fournie à l'étape (a).
